(19) Europäisches Patentamt
European Patent Office
Office européen des brevets

(11) EP 0 582 977 B1

(12) EUROPÄISCHE PATENTSCHRIFT

(45) Veröffentlichungstag und Bekanntmachung des Hinweises auf die Patenterteilung:
**27.11.1996 Patentblatt 1996/48**

(51) Int Cl.⁶: **C07D 209/34**

(21) Anmeldenummer: **93112623.9**

(22) Anmeldetag: **06.08.1993**

# (54) Verfahren zur Herstellung von 1,3-Dialkyl-5-hydroxyoxindolen und deren Etherderivaten

Process for the preparation of 1,3-dialkyl-5-hydroxyoxindoles and of their ether derivatives

Procédé pour la préparation de 1,3-dialkyl-5-hydroxyoxindoles et de leurs dérivés d'éthers

(84) Benannte Vertragsstaaten:
**AT BE CH DE DK ES FR GB GR IE IT LI LU NL PT SE**

(30) Priorität: **08.08.1992 DE 4226263**

(43) Veröffentlichungstag der Anmeldung:
**16.02.1994 Patentblatt 1994/07**

(73) Patentinhaber:
- **BOEHRINGER INGELHEIM KG**
  **D-55216 Ingelheim (DE)**
  Benannte Vertragsstaaten:
  **AT BE CH DE DK ES FR GB GR IE IT LI LU NL PT SE**
- **BOEHRINGER INGELHEIM INTERNATIONAL GmbH**
  **D-55216 Ingelheim am Rhein (DE)**
  Benannte Vertragsstaaten:
  **GB**

(72) Erfinder:
- **Sobotta, Rainer, Dr.**
  **D-55216 Ingelheim (DE)**
- **Schwarz, Rainer**
  **D-55216 Ingelheim (DE)**
- **Psiorz, Manfred, Dr.**
  **D-55216 Ingelheim (DE)**

(56) Entgegenhaltungen:
**DE-C- 335 763**

- **JOURNAL OF THE AMERICAN CHEMICAL SOCIETY, Bd. 57, Nr. 3, 1935, Seiten 563 - 566 P.L. JULIAN ET AL. 'Studies in ...'**
- **JOURNAL OF THE AMERICAN CHEMICAL SOCIETY, Bd. 96, Nr. 17, 1974, Seiten 5508 - 5512 P.G. GASSMAN ET AL. 'Oxindoles. ...'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 582 977 B1

**Beschreibung**

Die vorliegende Erfindung betrifft eine im technischen Maßstab durchführbare Herstellung von 1,3-Dialkyl-5-hydroxyoxindolen und deren Etherderivaten der allgemeinen Formel 1 durch Cyclisierung von N-Methyl-p-alkoxy-(2-halogenacyl)aniliden der allgemeinen Formel 2 gemäß folgendem Formelschema

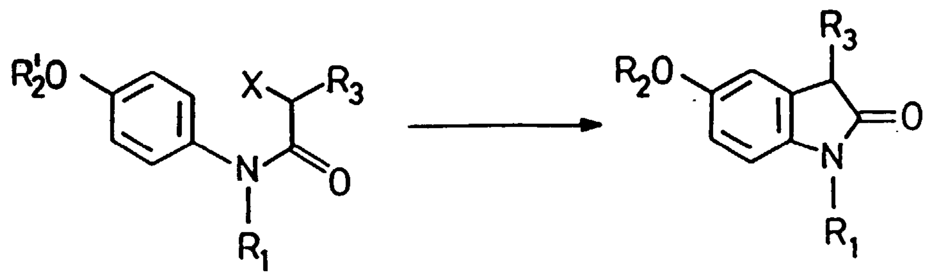

2 1

worin

$R_1$ $C_1$-$C_6$-Alkyl;
$R_2$ H, $C_1$-$C_6$-Alkyl;
$R_2$' $C_1$-$C_6$-Alkyl;
$R_3$ $C_1$-$C_6$-Alkyl und
X Chlor oder Brom

bedeuten.

$C_1$-$C_6$-Alkyl steht im allgemeinen für einen verzweigten oder unverzweigten Kohlenwasserstoffrest mit 1 bis 6 Kohlenstoffatom(en), der gegebenenfalls mit einem oder mehreren Halogenatom(en) - vorzugsweise Fluor - substituiert sein kann, die untereinander gleich oder verschieden sein können. Als Beispiele seien folgende Kohlenwasserstoffreste genannt: Methyl, Ethyl, Propyl, 1-Methylethyl (Isopropyl), Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl.
Bevorzugt sind - sofern nicht anders angegeben - Niederalkylreste mit 1 bis 4 Kohlenstoffatomen, wie z.B. Methyl, Ethyl, Propyl, Isopropyl, Butyl und tert.-Butyl.

Die Herstellung von 1,3-Dimethyl-5-hydroxy-oxindol bzw. von 1,3-Dimethyl-5-alkoxyindolen ist aus dem Stand der Technik bekannt. So beschreiben Pickl et al. [P.L. Julian und J. Pikl, J. Am. Chem. Soc. 57 (1935) 563] ein Verfahren zur Herstellung von 1,3-Dimethyl-5-hydroxy-oxindol, welches diese Verbindung durch Cyclisierung von N-Methyl-p-ethoxy-α-brompropionylanilid mittels wasserfreiem Aluminiumchlorid im Labormaßstab verfügbar macht.

Daneben ist aus der deutschen Patentschrift 335 763 ein Verfahren zur Herstellung von N-substituierten Oxindolen beschrieben. Bei dem dort beschriebenen Verfahren werden N-Monohalogenacylderivate von sekundären Alkylarylaminen in Gegenwart von Aluminiumhalogeniden auf Temperaturen im Bereich von 160 bis 180°C erhitzt, wobei unter Halogenwasserstoffabspaltung die Cyclisierung zum entsprechenden N-substituierten Oxindol-Derivat erfolgt.

Des weiteren beschreiben P. G. Gassman und T. J. van Bergen [J. Am.Chem. Soc. 96 (1974) 5508] eine Synthese zur Herstellung von Oxindolen bei dem geeignete Anilinderivate in Gegenwart von tert.-Butylhypochlorit und einer Base mit β-Ketosulfiden - wie z.B. Ethylmethylthioacetat - umgesetzt werden.

Durch das Erhitzen der zunächst gebildeten α-Aminoaryl-α-alkylthioacetate in saurem Medium werden zunächst die entsprechenden 3-Alkylthiooxindolderivate zugänglich, die auf dem Wege der Desulfurierung in die gewünschten Oxindolderivate überführt werden.

Für eine industrielle Anwendung sind diese Verfahren jedoch nicht geeignet, da sie - sofern Aluminium(III)chlorid als Katalysator eingesetzt wird - folgende Nachteile in sich bergen:

- der Katalysator Aluminium(III)chlorid muß mindestens in einem 6,7-fachen Überschuß pro Mol Edukt eingesetzt werden,

- die Hydrolyse des Aluminium(III)chlorids stellt eine stark exotherme Reaktion dar,

- da Aluminium(III)chlorid zur Sublimation neigtt, sublimiert es beim Erwärmen der Reaktionsmischung in den Apparateaufbau, wonach es für die Cyclisierung nicht mehr zur Verfügung steht,

- mit der Verwendung von Aluminium(III)chlorid als Katalysator ist die Gefahr von Folge- und Nebenraktionen verbunden, die bei höheren Temperaturen für eine Etherspaltung verantwortlich sind, wodurch zur Herstellung eines gegebenenfalls gewünschten Oxindolethers in der weiteren Reaktionsfolge einen weiteren Alkylierungsschritt erforderlich macht,

- die quantitative Abtrennung des aus der Hydrolyse resultierenden Aluminiumhydroxids bereitet Schwierigkeiten.

Das - mehrstufige - von P. Gassman beschriebene Verfahren leidet zwar nicht unter den oben angeführten Nachteilen, da es ohne den Einsatz Aluminium-Katalysatoren auskommt. Auf der anderen Seite ist in der abschließenden Stufe eine reduktive Spaltung der aus der Cyclisierungsreaktion unnmittelbar resultierenden Thioethergruppierung mit Raney-Nickel als Desulfurierungsreagenz erforderlich.

Gegenstand der vorliegenden Erfindung ist somit, ein industriell bzw. großtechnisch anwendbares Verfahren zur Herstellung von 1,3-Dialkyl-5-hydroxindolen und seinen Etherderivaten gemäß der allgemeinen Formel (1) vorzuschlagen, welches die aus dem Stand der Technik bekannten Nachteile überwindet.

Erfindungsgemäß wird diese Aufgabe dadurch gelöst,daß man N-Alkyl-p-alkoxy-($\alpha$-halogenacylanilide) der allgemeinen Formel 2

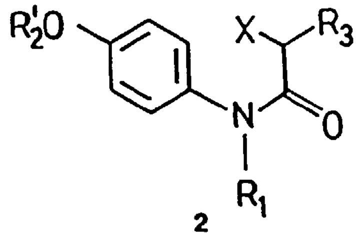

**2**

worin

$R_1$ $C_1$-$C_6$-Alkyl;
$R_2'$ $C_1$-$C_6$-Alkyl;
$R_3$ $C_1$-$C_6$-Alkyl und
X Chlor oder Brom

bedeuten,
mit 1 bis 2.5 Mol (die Mol-Angaben beziehen sich jeweils auf ein Mol des eingesetzten Anilids vom Typ 2) - bevorzugt 1.75 bis 2 Mol - eines wasserfreien Zinkhalogenids - bevorzugt Zinkchlorid oder Zinkbromid - versetzt und bei einer Temperatur im Bereich von 120 bis 180°C, bevorzugt 130 bis 175°C und besonders bevorzugt in einem Bereich von 145 bis 160°C zur Reaktion bringt. Die Reaktionszeit richtet sich danach, ob die Reaktion in Substanz oder in einem Lösungsmittel durchgeführt wird. In ersterem Fall liegen die Reaktionszeiten in einem Intervall von 1 bis 7, bevorzugt 2 bis 5 und besonders bevorzugt im Bereich von 3 bis 4 Stunden. Bei der Verwendung von Lösungsmitteln verlängern sich die Reaktionszeiten auf 7 bis 20 bevorzugt 8 bis 16 und besonders bevorzugt 10 bis 16 Stunden. Das Reaktionsgemisch wird nach beendeter Reaktion hydrolysiert und das so hergestellte Oxindolderivat der allgemeinen Formel 1 ($R_2$ = H) isoliert.

Zur Herstellung der verätherten Derivate ($R_2$ = $C_1$-$C_6$-Alkyl) ist es lediglich notwendig, ein inertes Lösungsmittel, das auf die Cyclisierungsreaktion keinen nachteiligen Einfluß ausübt, einzusetzen. Bevorzugt werden also unter den angewandten Reaktionsbedingungen inerte Lösungsmittel, worunter Chlorbenzol und Toluol besonders bevorzugt werden, eingesetzt.

Durch Zusatz von 1 Mol einer organischen Base pro Mol N-Methyl-p-alkoxy-($\alpha$-halogenpropionyl)-anilid bevorzugt im Gemisch mit Zinkhalogenid und Lösungsmittel kann die Ätherspaltung nahezu vollständig unterdrückt werden. Bevorzugt werden 2 bis 3 Mol Zinkchlorid oder Zinkbromid pro Mol N-Methyl-p-alkoxy-($\alpha$-halogenpropionyl)-anilid eingesetzt. Die Reaktionszeiten verlängern sich dabei und liegen in einem Bereich von 15 bis 30, bevorzugt 16 bis 24 Stunden. Hierzu werden bevorzugt organische Stickstoffbasen verwandt, besonders bevorzugt Trialkylamine - wie z. B. Triethylamin - N,N-Dialkylaniline sowie heterocyclische Basen - wie z.B. Pyridin oder Chinolin.

Die eingangs genannte Aufgabe wird insbesondere durch die nachfolgend aufgeführten Beispiele gelöst. Verschiedenartige, andere und weitere Merkmale, Ausgestaltungen des Verfahrens und dergleichen, die der vorliegenden Erfindung zugeordnet sind, werden für den Fachmann aus der vorliegenden Beschreibung ersichtlich und in Verbindung mit den Beispielen, in welchen die gegenwärtig bevorzugten Ausführungsformen der Erfindung beispielhaft dargestellt sind, noch besser verständlich. - Es wird jedoch ausdrücklich darauf hingewiesen, daß die Beispiele und die diesen zugeordnete Beschreibung lediglich zum Zweck der Erläuterung und Beschreibung vorgesehen und nicht als Einschränkung der Erfindung anzusehen sind.

**Beispiel 1**

In einem 250 ml Dreihalskolben mit mechanischem Rührer und Gasableitungsrohr zum Gaswäscher werden 75,6 g (0,25 Mol) N-Methyl-p-methoxy-(α-brompropionyl)anilid und 59,6 g (0,437 Mol) wasserfreies Zinkchlorid eingefüllt. Unter Rühren wird die Temperatur erhöht, wobei die Reaktionsmasse zunehmend dünnflüssiger wird. Ab einer Temperatur von 145°C beginnt eine spontane Gasentwicklung und die Sumpftemperatur steigt kurzzeitig auf 160°C. Beim nachfolgenden Absinken der Temperatur wird das Reaktionsgemisch durch Heizen drei Stunden auf einer Temperatur von 160°C gehalten. Danach werden innerhalb von 5 Minuten 50 ml Wasser zudosiert und das Gemisch anschließend in 300 ml Wasser eingegossen. Nach zehnminütigem Rühren und Abkühlen des Gemisches auf 20°C werden die dabei entstandenen Kristalle aus der Suspension abfiltriert und getrocknet. Man erhält 41,0 g (92,55 % d. Th.) dunkelgrau gefärbtes 1,3-Dimethyl-5-hydroxy-oxindol.

**Beispiel 2**

Die Durchführung erfolgt gemäß Beispiel 1; das Reaktionsgemisch aus 226,8 g (0,75 Mol) N-Methyl-p-methoxy-(α-brompropionyl)anilid und 178,9 (1,31 Mol) Zinkchlorid wird unter Rühren auf 65°C aufgeheizt bis es gut fließfähig ist. Anschließend wird eine Teilmenge (ca. 20 %) in ein darunterliegendes auf 160°C vorgeheiztes 750 ml-Reaktionsgefäß (mit Rührer und Gasableitung) dosiert. Nach dem Einsetzen der Reaktion (ab 145°C) und Abklingen der spontanen Gasentwicklung wird die Hauptmenge des Reaktionsgemisches aus dem oberen Gefäß innerhalb 70 Minuten zudosiert. Insgesamt wird das Reaktionsgemisch drei Stunden auf 160°C erwärmt. Die Aufarbeitung erfolgt gemäß Beispiel 1.
Ausbeute: 124,8 g (93,9 % d. Th.) an rohem 1,3-Dimethyl-5-hydroxy-oxindol.

Die Umkristallisation von 100 g Rohware erfolgt aus 1480 ml Methanol. Unter Zusatz von 5,4 g Aktivkohle wird heiß gelöst und filtriert. Nach dem Einengen des Filtrats auf 150 ml wird dieses über einen Zeitraum von ca. 12 Stunden auf eine Temperatur von -25°C abgekühlt. Die dabei abgeschiedenen Kristalle werden abgesaugt und getrocknet. Man gewinnt auf diesem Wege das 1,3-Dimethyl-5-hydroxyoxindol in einer Ausbeute von 69,5 g (69,5 % vom Einsatz) in Form von Kristallen mit einem Schmelzpunkt von 207-209°C.

**Beispiel 3**

Die Durchführung erfolgt auch in diesem Beispiel gemäß Beispiel 1: Die Gesamtmenge Zinkchlorid (28.6 g; 0,21 Mol) wird mit der Teilmenge (ca. 20 %) des N-Methyl-p-methoxy-(α-brompropionyl)anilid (6,5 g; 0,024 Mol) auf eine Temperatur von 145°C-160°C erhitzt. Nach dem Abklingen der Gasentwicklung wird die Hauptmenge des Anilids (26,1 g; 0,096 Mol) innerhalb von 60 Minuten zu dem Reaktionsgemisch zudosiert und insgesamt drei Stunden auf eine Temperatur von 160°C erwärmt. Die Aufarbeitung erfolgt wie in Beispiel 1 angegeben. Man isoliert auf diese Weise das 1,3-Dimethyl-5-hydroxy-oxindol einer Rohausbeute von 18.22 g (85,7 % d. Th.).

**Beispiel 4**

Analog Beispiel 1: 14,95 g (0,05 Mol) N-Methyl-p-ethoxy-(α-brompropionyl)anilid werden 90 Minuten auf eine Temperatur von 160-165°C erhitzt und anschließend gemäß Beispiel 1 aufgearbeitet. Ausbeute: 8,2 g (92,5 % d. Th.) an rohem 1,3-Dimethyl-5-hydroxy-oxindol.

**Beispiel 5**

Die Durchführung erfolgt analog Beispiel 1: 38,2 g (0,15 Mol) N-Methyl-p-ethoxy-(α-chlorpropionyl)-anilid und 35,77 g (0,2625 Mol) Zinkchlorid werden unter Rühren erhitzt. Die Reaktion beginnt bei 135°C. Die Reaktionstemperatur wird über einen Zeitraum von 110 Minuten bei 145-150°C gehalten. Nach Aufarbeitung analog Beispiel 1 werden 24,2 g (91,0 % d. Th.) an rohem 1,3-Dimethyl-5-hydroxy-oxindol isoliert.

**Beispiel 6**

14,96 g (0,05 Mol) N-Methyl-p-ethoxy-(α-brompropionyl)-α-anilid und 13,63 g (0,1 Mol) wasserfreies Zinkchlorid in 100 ml Chlorbenzol werden unter Rühren und Rückfluß 10 Stunden auf Rückflußtemperatur erhitzt. Nach dem Abkühlen auf 50°C wird das Reaktionsgemisch zweimal mit je 20 ml Wasser ausgerührt. Anschließend wird die organische Phase zweimal mit je 40 ml verdünnter Kalilauge (2,5 %-ig) und mit 30 ml Wasser ausgerührt. Die organische Phase wird isoliert und zum Rückstand eingeengt. Die Kristallisation des Rückstands (7,1 g) erfolgt aus einem Gemisch von 30 ml Cyclohexan und 2 ml Aceton. Man erhält 5,58 g (54,3 % d. Th.) 1,3-Dimethyl-5-ethoxy-oxindol.

Die vereinigten alkalischen wäßrigen Phasen werden mit Schwefelsäure versetzt. Aus der sauren Lösung (pH 1) scheidet sich ein kristalliner Niederschlag ab. Nach Trocknung werden 1,76 g (19,9 % d. Th.) 1,3-Dimethyl-5-hydroxy-oxindol erhalten.

**Beispiel 7**

Die Durchführung erfolgt analog Beispiel 6: 12,73 g (0,05 Mol) N-Methyl-p-ethoxy-(α-chlorpropionyl)anilid und 13,63 g (0,1 Mol) Zinkchlorid in 20 ml Chlorbenzol werden unter Rühren und Rückfluß 8 Stunden auf Rückflußtemperatur erhitzt. Nach Aufarbeitung werden - wie in Beispiel 6 angegeben -
3,16 (33,9 % d. Th.) 1,3-Dimethyl-5-ethoxy-oxindol und
2,78 g (31,4 % d. Th.) 1,3-Dimethyl-5-hydroxy-oxindol erhalten.

**Beispiel 8**

Die Durchführung erfolgt analog Beispiel 6: 7,45 g (0,025 Mol) N-Methyl-p-ethoxy-(α-brom-propionyl)anilid 10,2 g (0,075 Mol) wasserfreies Zinkchlorid und 2,53 g (0,025 Mol) Triethylamin in 50 ml Chlorbenzol werden unter Rühren und Rückfluß 24 Stunden auf Rückflußtemperatur erhitzt. Nach dem Abkühlen wird wie in Beispiel 6 angegeben, aufgearbeitet. Zur vollständigen Abtrennung des Triethylamins wird die erste Wassermenge zusätzlich mit Salzsäure angesäuert.
Man erhält 4,05 g (79 % d. Th.) an rohem 1,3-Dimethyl-5-ethoxy-oxindol.

**Beipiel 9**

Die Durchführung erfolgt analog Beispiel 8: 7,45 g (0,025 Mol) N-Methyl-p-ethoxy-(α-brompropionyl)-anilid 16,9 g (0,075 Mol) wasserfreies Zinkbromid und 2,59 g (0,025 Mol) Triethylamin in 40 ml Chlorbenzol werden unter Rühren und Rückfluß 24 Stunden auf Rückflußtemperatur erhitzt. Nach Aufarbeitung wie in Beispiel 8 erhält man 4,79 g (93,4 % d. Th.) an rohem 1,3-Dimethyl-5-ethoxy-oxindol.

**Patentansprüche**

1. Verfahren zur Herstellung von 1,3-Dialkyl-5-hydroxy-oxindolen der allgemeinen Formel 1,

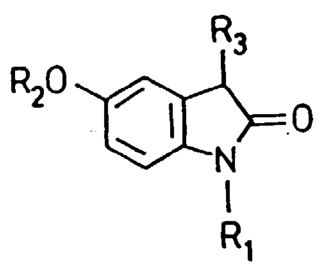

**1**

worin

$R_1$ $C_1$-$C_6$-Alkyl;
$R_2$ Wasserstoff und
$R_3$ $C_1$-$C_6$-Alkyl

bedeuten, dadurch gekennzeichnet, daß man N-Alkyl-p-alkoxy-(α--halogenacyl)anilide der allgemeinen Formel 2,

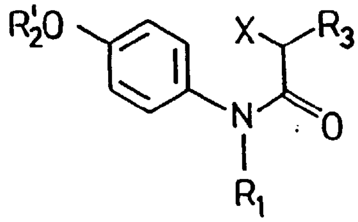

2

worin

$R_1$ $C_1$-$C_6$-Alkyl;
$R'_2$ $C_1$-$C_6$-Alkyl;
$R_3$ $C_1$-$C_6$-Alkyl und
X Chlor oder Brom

bedeuten in Gegenwart von 1 bis 2.5 Mol eines wasserfreien Zinkhalogenids auf eine Temperatur im Bereich von 120 bis 180°C erhitzt, die Reaktionsmischung nach beendeter Cyclisierung wässerig aufarbeitet und das Oxindolderivat der allgemeinen Formel 1 isoliert.

**2.** Verfahren nach Anspruch 1, dadurch gekennzeichnet, daß man N-Alkyl-p-alkoxy-(α-halogenacyl)anilide der allgemeinen Formel 2 in Gegenwart von 1.75 bis 2.0 Mol Zinkbromid und/oder Zinkchlorid auf eine Temperatur im Bereich von 130 bis 175°C erhitzt, die Reaktionsmischung nach beendeter Cyclisierung wässerig aufarbeitet und das Oxindolderivat der allgemeinen Formel 1 isoliert.

**3.** Verfahren nach Anspruch 1, dadurch gekennzeichnet, daß man N-Alkyl-p-alkoxy-(α-halogenacylanilide) der allgemeinen Formel 2 in Gegenwart von 1.75 bis 2.0 Mol Zinkchlorid und/oder Zinkbromid auf eine Temperatur im Bereich von 145 bis 160°C erhitzt, die Reaktionsmischung nach beendeter Cyclisierung wässerig aufarbeitet und das Oxindolderivat der allgemeinen Formel I isoliert.

**4.** Verfahren zur Herstellung von 1,3-Dialkyl-5-alkoxy-oxindolen der allgemeinen Formel 1

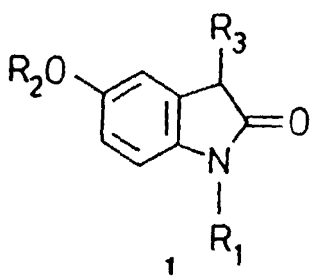

1

worin

$R_1$ $C_1$-$C_6$-Alkyl;
$R_2$ $C_1$-$C_6$-Alkyl und
$R_3$ $C_1$-$C_6$-Alkyl

bedeuten, dadurch gekennzeichnet, daß man N-Alkyl-p-alkoxy-(α-halogenacyl)anilide der allgemeinen Formel 2,

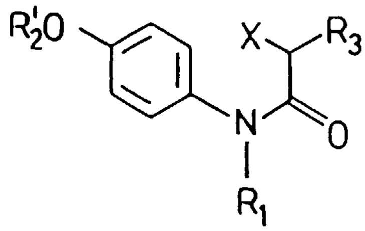

2

worin

$R_1$ $C_1$-$C_6$-Alkyl;
$R_2'$ $C_1$-$C_6$-Alkyl
$R_3$ $C_1$-$C_6$-Alkyl und
X Chlor oder Brom

bedeuten, in einem inerten gegebenenfalls polaren, Lösungsmittel in Gegenwart eines wasserfreien Zinkhalogenids und gegebenenfalls einer organischen Base auf eine Temperatur im Bereich von 120 bis 180°C erhitzt, die Reaktionsmischung nach beendeter Cyclisierung wässerig aufarbeitet und das 1,3-Dialkyl-5-alkoxyindolderivat der allgemeinen Formel 1 isoliert.

**5.** Verfahren nach Anspruch 4, dadurch gekennzeichnet, daß man N-Allkyl-p-alkoxy-(α-halogenacyl)anilide der allgemeinen Formel 2 in einem inerten Lösungsmittel in Gegenwart von 1.0 bis 2.5 Mol wasserfreien Zinkchlorids auf eine Temperatur im Bereich von 130 bis 175°C erhitzt, die Reaktionsmischung nach beendeter Cyclisierung wässerig aufarbeitet und das 1,3-Dialkyl-5-alkoxyindolderivat der allgemeinen Formel 1 isoliert.

**6.** Verfahren nach Anspruch 4, dadurch gekennzeichnet, daß man N-Alkyl-p-alkoxy-(α-halogenacyl)-anilide der allgemeinen Formel 2 in Chlorbenzol in Gegenwart von 1.75 bis 2.0 Mol wasserfreien Zinkchlorids auf eine Temperatur im Bereich von 145 bis 160°C erhitzt, die Reaktionsmischung nach beendeter Cyclisierung wässerig aufarbeitet und das 1,3-Dialkyl-5-alkoxyindolderivat der allgemeinen Formel 1 isoliert.

**7.** Verfahren nach einem der Ansprüche 4 oder 6, dadurch gekennzeichnet, daß man als inertes Lösungsmittel Chlorbenzol oder Toluol einsetzt.

**8.** Verfahren nach Anspruch 4 bis 7, dadurch gekennzeichnet, daß man N-Alkyl-p-alkoxy-(α-halogenacyl)anilide der allgemeinen Formel 2 in einem inerten Lösungsmittel in Gegenwart von 2.0 bis 3.0 Mol wasserfreien Zinkchlorids und/oder Zinkbromids und einer organischen Stickstoffbase auf eine Temperatur im Bereich von 130 bis 175°C erhitzt, die Reaktionsmischung nach beendeter Cyclisierung wässerig aufarbeitet und das 1,3-Dialkyl-5-alkoxyindolderivat der allgemeinen Formel 1 isoliert.

**9.** Verfahren nach einem der Ansprüche 4 bis 8, dadurch gekennzeichnet, daß man N-Alkyl-p-alkoxy-(α-halogenacyl)-anilide der allgemeinen Formel 2 in Chlorbenzol oder Toluol in Gegenwart von 2.0 bis 3.0 Mol wasserfreien Zinkchlorids und/oder Zinkbromids und in Gegenwart von 1.0 Mol eines Trialkylamins und/oder eines N,N-Dialkylanilins und/oder Pyridin und/oder Chinolin auf eine Temperatur im Bereich von 145 bis 160°C erhitzt, die Reaktionsmischung nach beendeter Cyclisierung wässerig aufarbeitet und das 1,3-Dialkyl-5-alkoxyindolderivat der allgemeinen Formel 1 isoliert.

**10.** Verfahren nach Anspruch 9, dadurch gekennzeichnet, daß man als Trialkylamin Triethylamin einsetzt.

**Claims**

**1.** Process for preparing 1,3-dialkyl-5-hydroxyoxindoles of general formula 1

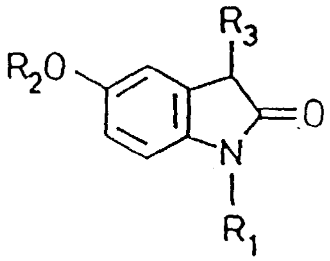

1

wherein

$R_1$ denotes $C_1$-$_6$-alkyl;
$R_2$ denotes hydrogen and
$R_3$ denotes $C_{1-6}$-alkyl,

characterised in that N-alkyl-p-alkoxy-(α-haloacyl)anilides of general formula 2

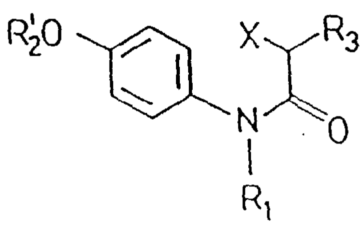

2

wherein

$R_1$ denotes $C_{1-6}$-alkyl,
$R'_2$ denotes $C_{1-6}$-alkyl;
$R_3$ denotes $C_{1-6}$-alkyl and
X denotes chlorine or bromine

is heated in the presence of 1 to 2.5 mol of an anhydrous zinc halide to a temperature in the range from 120 to 180°C, after cyclisation has ended the reaction mixture is worked up under aqueous conditions and the oxindole derivative of general formula 1 is isolated.

**2.** Process according to claim 1, characterised in that N-alkyl-p-alkoxy-(α-haloacyl)anilides of general formula 2 are heated, in the presence of 1.75 to 2.0 mol of zinc bromide and/or zinc chloride, to a temperature in the range from 130 to 175°C, after cyclisation has ended the reaction mixture is worked up under aqueous conditions and the oxindole derivative of general formula 1 is isolated.

**3.** Process according to claim 1, characterised in that N-alkyl-p-alkoxy-(α-haloacylanilides) of general formula 2 are heated in the presence of 1.75 to 2.0 mol of zinc chloride and/or zinc bromide to a temperature in the range from 145 to 160°C, after cyclisation has ended the reaction mixture is worked up under aqueous conditions and the oxindole derivative of general formula 1 is isolated.

**4.** Process for preparing 1,3-dialkyl-5-alkoxyindoles of general formula 1

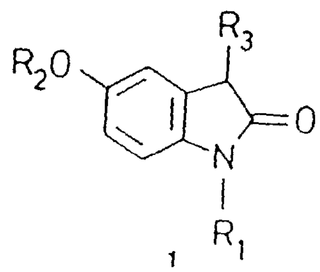

1

wherein

$R_1$ denotes $C_{1-6}$-alkyl;
$R_2$ denotes $C_{1-6}$-alkyl and
$R_3$ denotes $C_{1-6}$-alkyl,

characterised in that N-alkyl-p-alkoxy-(α-haloacyl)anilides of general formula 2

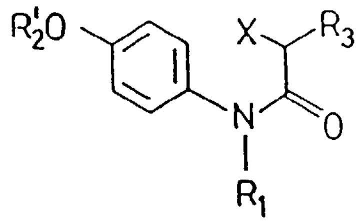

2

wherein

$R_1$ denotes $C_{1-6}$-alkyl;
$R'_2$ denotes $C_{1-6}$-alkyl;
$R_3$ denotes $C_{1-6}$-alkyl and
X denotes chlorine or bromine

are heated in an inert, optionally polar, solvent in the presence of an anhydrous zinc halide and optionally an organic base to a temperature in the range from 120 to 180°C, after cyclisation has ended the reaction mixture is worked up under aqueous conditions and the 1,3-dialkyl-5-alkoxyindole derivative of general formula 1 is isolated.

**5.** Process according to claim 4, characterised in that N-alkyl-p-alkoxy-(α-haloacyl)anilides of general formula 2 are heated to a temperature in the range from 130 to 175°C in an inert solvent in the presence of 1.0 to 2.5 mol of anhydrous zinc chloride, after cyclisation has ended the reaction mixture is worked up under aqueous conditions and the 1,3-dialkyl-5-alkoxyindole derivative of general formula 1 is isolated.

**6.** Process according to claim 4, characterised in that N-alkyl-p-alkoxy-(α-haloacyl)anilides of general formula 2 are heated to a temperature in the range from 145 to 160°C in chlorobenzene in the presence of 1.75 to 2.0 mol of anhydrous zinc chloride, after cyclisation has ended the reaction mixture is worked up under aqueous conditions and the 1,3-dialkyl-5-alkoxyindole derivative of general formula 1 is isolated.

**7.** Process according to one of claims 4 or 6, characterised in that chlorobenzene or toluene is used as an inert solvent.

**8.** Process according to claims 4 to 7, characterised in that N-alkyl-p-alkoxy-(α-haloacyl)-anilides of general formula 2 are heated to a temperature in the range from 130 to 175°C in an inert solvent in the presence of 2.0 to 3.0 mol of anhydrous zinc chloride and/or zinc bromide and an organic nitrogen base, after cyclisation has ended the reaction mixture is worked up under aqueous conditions and the 1,3-dialkyl-5-alkoxyindole derivative of general formula 1 is isolated.

**9.** Process according to one of claims 4 to 8, characterised in that N-alkyl-p-alkoxy-(α-haloacyl)-anilides of general formula 2 are heated to a temperature in the range from 145 to 160°C in chlorobenzene or toluene in the presence of 2.0 to 3.0 mol of anhydrous zinc chloride and/or zinc bromide and 1.0 mol and in the presence of a trialkylamine and/or an N,N-dialkylaniline and/or pyridine and/or quinoline, after cyclisation has ended the reaction mixture is worked up under aqueous conditions and the 1,3-dialkyl-5-alkoxyindole derivative of general formula 1 is isolated.

**10.** Process according to claim 9, characterised in that triethylamine is used as the trialkylamine.

## Revendications

**1.** Procédé de préparation de 1,3-dialkyl-5-hydroxyoxindoles de formule générale 1

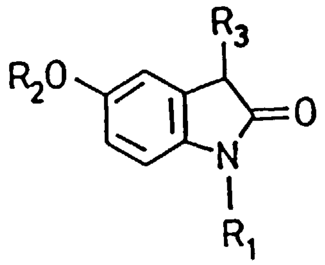

**1**

dans laquelle

$R_1$ représente un alkyle en $C_1$-$C_6$,
$R_2$ représente l'hydrogène et
$R_3$ représente un alkyle en $C_1$-$C_6$

caractérisé en ce que l'on chauffe des N-alkyl-p-alcoxy-(α-halogénoacyl)anilides de formule générale 2

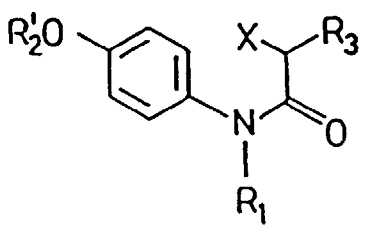

**2**

dans laquelle

$R_1$ représente un alkyle en $C_1$-$C_6$,
$R'_2$ représente un alkyle en $C_1$-$C_6$,
$R_3$ représente un alkyle en $C_1$-$C_6$ et
X représente le chlore ou le brome

en présence de 1 à 2,5 moles d'un halogénure de zinc anhydre à une température située dans le domaine de 120 à 180°C, après la fin de la cyclisation on traite le mélange réactionnel en milieu aqueux et on isole le dérivé d'oxindole de formule générale 1.

**2.** Procédé selon la revendication 1, caractérisé en ce que l'on chauffe des N-alkyl-p-alcoxy-(α-halogénoacyl)-anilides de formule générale 2 en présence de 1,75 à 2,0 moles de bromure de zinc et/ou de chlorure de zinc à une température située dans le domaine de 130 à 175°C, après la fin de la cyclisation on traite le mélange réactionnel en milieu aqueux et on isole le dérivé d'oxindole de formule générale 1.

**3.** Procédé selon la revendication 1, caractérisé en ce que l'on chauffe des N-alkyl-p-alcoxy-(α-halogénoacyl)-anilides de formule générale 2 en présence de 1,75 à 2,0 moles de chlorure de zinc et/ou de bromure de zinc à une température située dans le domaine de 145 à 160°C, après la fin de la cyclisation on traite le mélange réactionnel en milieu aqueux et on isole le dérivé d'oxindole de formule générale 1.

**4.** Procédé de préparation de 1,3-dialkyl-5-alcoxyoxindoles de formule générale 1

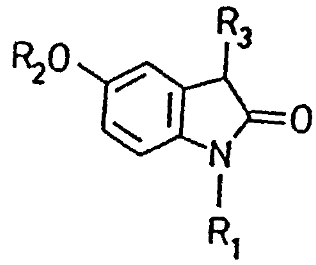

**1**

dans laquelle

$R_1$ représente un alkyle en $C_1$-$C_6$,
$R_2$ représente un alkyle en $C_1$-$C_6$ et
$R_3$ représente un alkyle en $C_1$-$C_6$

caractérisé en ce que l'on chauffe des N-alkyl-p-alcoxy-(α-halogénoacyl)anilides de formule générale 2

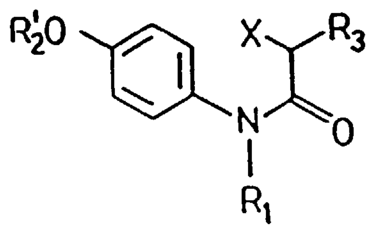

**2**

dans laquelle

$R_1$ représente un alkyle en $C_1$-$C_6$,
$R'_2$ représente un alkyle en $C_1$-$C_6$,
$R_3$ représente un alkyle en $C_1$-$C_6$ et
X représente le chlore ou le brome

dans un solvant inerte éventuellement polaire, en présence d'un halogénure de zinc anhydre et éventuellement d'une base organique à une température située dans le domaine de 120 à 180°C, après la fin de la cyclisation on traite le mélange réactionnel en milieu aqueux et on isole le dérivé de 1,3-dialkyl-5-alcoxyindole de formule générale 1.

**5.** Procédé selon la revendication 4, caractérisé en ce que l'on chauffe des N-alkyl-p-alcoxy-(α-halogénoacyl)-anilides de formule générale 2 dans un solvant inerte en présence de 1,0 à 2,5 moles de chlorure de zinc anhydre à une température située dans le domaine de 130 à 175°C, après la fin de la cyclisation on traite le mélange réactionnel en milieu aqueux et on isole le dérivé de 1,3-dialkyl-5-alcoxyindole de formule générale 1.

**6.** Procédé selon la revendication 4, caractérisé en ce que l'on chauffe des N-alkyl-p-alcoxy-(α-halogénoacyl)-anilides de formule générale 2 dans le chlorobenzène en présence de 1,75 à 2,0 moles de chlorure de zinc anhydre à une température située dans le domaine de 145 à 160°C, après la fin de la cyclisation on traite le mélange réactionnel

en milieu aqueux et on isole le dérivé de 1,3-dialkyl-5-alcoxyindole de formule générale 1.

7. Procédé selon l'une des revendications 4 et 6, caractérisé en ce que l'on utilise le chlorobenzène ou le toluène comme solvant inerte.

8. Procédé selon les revendications 4 à 7, caractérisé en ce que l'on chauffe des N-alkyl-p-alcoxy-(α-halogénoacyl) anilides de formule générale 2 dans un solvant inerte en présence de 2,0 à 3,0 moles de chlorure de zinc et/ou de bromure de zinc anhydre et d'une base azotée organique à une température située dans le domaine de 130 à 175°C, après la fin de la cyclisation on traite le mélange réactionnel en milieu aqueux et on isole le dérivé de 1,3-dialkyl-5-alcoxyindole de formule générale 1.

9. Procédé selon l'une des revendications 4 à 8, caractérisé en ce que l'on chauffe des N-alkyl-p-alcoxy-(α-halogéno-acyl)anilides de formule générale 2 dans le chlorobenzène ou le toluène en présence de 2,0 à 3,0 moles de chlorure de zinc et/ou de bromure de zinc anhydre et de 1,0 mole et en présence d'une trialkylamine et/ou d'une N,N-dialkylaniline et/ou de pyridine et/ou de quinoléine à une température située dans le domaine de 145 à 160°C, après la fin de la cyclisation on traite le mélange réactionnel en milieu aqueux et on isole le dérivé de 1,3-dialkyl-5-alcoxyindole de formule générale 1.

10. Procédé selon la revendication 9, caractérisé en ce que l'on utilise la triéthylamine comme trialkylamine.